(19) Europäisches Patentamt / European Patent Office / Office européen des brevets

(11) **EP 3 493 861 B1**

(12) **EUROPÄISCHE PATENTSCHRIFT**

(45) Veröffentlichungstag und Bekanntmachung des Hinweises auf die Patenterteilung:
**03.05.2023 Patentblatt 2023/18**

(21) Anmeldenummer: **17752286.9**

(22) Anmeldetag: **03.08.2017**

(51) Internationale Patentklassifikation (IPC):
**A61M 1/16** *(2006.01)* **A61K 33/00** *(2006.01)*

(52) Gemeinsame Patentklassifikation (CPC):
**A61M 1/1607; A61K 33/00; A61M 1/1613; A61M 1/165; A61M 1/1656;** A61M 2205/3317

(86) Internationale Anmeldenummer:
**PCT/EP2017/000942**

(87) Internationale Veröffentlichungsnummer:
**WO 2018/024373 (08.02.2018 Gazette 2018/06)**

(54) **VERFAHREN ZUR ÜBERWACHUNG DES BICARBONAT-GEHALTS UND DES NATRIUM-GEHALTS EINER DIALYSELÖSUNG**

METHOD OF MONITORING THE BICARBONATE CONTENT AND THE SODIUM CONTENT OF A DIALYSIS SOLUTION

PROCÉDÉ POUR SURVEILLER LA TENEUR EN BICARBONATE ET LA TENEUR EN SODIUM D'UN DIALYSAT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.08.2016 DE 102016009442**

(43) Veröffentlichungstag der Anmeldung:
**12.06.2019 Patentblatt 2019/24**

(73) Patentinhaber: **Fresenius Medical Care Deutschland GmbH**
**61352 Bad Homburg (DE)**

(72) Erfinder:
• **GAGEL, Alfred**
**96123 Litzendorf (DE)**
• **STÄBLEIN, Tilman**
**97072 Würzburg (DE)**
• **POPP, Jochen**
**97464 Oberwerrn (DE)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(56) Entgegenhaltungen:
EP-A2- 0 311 848　　EP-A2- 0 597 817
US-A1- 2014 319 030　　US-A1- 2016 051 949

## Beschreibung

Die vorliegende Erfindung betrifft ein Verfahren zur Überwachung des Bicarbonat-Gehalts sowie des Natrium-Gehalts einer Dialyselösung, wobei die Dialyselösung unter Zugabe einer Bicarbonat-Komponente sowie einer sauren Natriumkomponente hergestellt wird.

Ein wichtiges Behandlungsverfahren bei chronischem Nierenversagen ist die Hämodialyse. Bei der Hämodialyse wird das zu reinigende Blut des Patienten auf einer Seite einer Membran eines Dialysators entlanggeführt. Auf der anderen Seite der Membran befindet sich die Dialyselösung, in die aufgrund eines Konzentrationsgradienten zwischen dem Blut und der Dialyselösung Inhaltsstoffe aus dem Blut über die Membran diffundieren. Um zu verhindern, dass dem Patienten während der Behandlung kontinuierlich lebensnotwendige Stoffe entzogen werden, ist es erforderlich, die Dialyselösung mit diesen Stoffen in physiologischen Konzentrationen zu versehen. Dabei sind neben den Elektrolyten Calcium, Magnesium und Kalium vor allem Natrium und Bicarbonat (Hydrogencarbonat) ausschlaggebend.

Aus dem Stand der Technik ist es bekannt, die benötigte Dialyselösung aus zwei Konzentraten unter Zugabe von Wasser herzustellen. Dabei beinhaltet ein Konzentrat hauptsächlich Natrium, während das andere Konzentrat vorwiegend Bicarbonat enthält.

Die Dialysenorm IEC 60601-2-16 (3rd und 4th Edition) fordert ein unabhängiges Schutzsystem, das eine Gefährdung von Patienten aufgrund einer fehlerhaften Zusammensetzung der Dialyselösung verhindert.

Aus der EP 0 597 817 B1 ist ein Dialysegerät bekannt, das eine Zubereitungseinrichtung für die Dialyselösung aufweist. Die Zubereitungseinrichtung weist eine mit einer Wasserquelle in Verbindung stehende Hauptleitung auf, in die eine erste und eine zweite Zuleitung münden, wobei die erste Zuleitung mit einem Natrium-Konzentratbehälter und die zweite Zuleitung mit einem Bicarbonat-Konzentratbehälter in Verbindung steht. Über Pumpen werden der durch die Hauptleitung strömenden Flüssigkeit die Konzentrate zugemischt, um eine gebrauchsfertige Dialyselösung zu erhalten. In der Hauptleitung ist stromabwärts der Einmündung des Natrium-haltigen Konzentrats eine Leitfähigkeitsmesszelle angeordnet. Eine weitere Leitfähigkeitsmesszelle befindet sich in der Hauptleitung stromabwärts der Einmündung des Bicarbonat Konzentrats. Eine dritte Leitfähigkeitsmesszelle ist zwischen der Bilanzierkammer und dem Dialysator angeordnet.

Ein derartiges Dialysegerät ist mit dem Nachteil eines vergleichsweise komplexen Aufbaus behaftet.

Die US2014/319030A1 offenbart Dialysatformulierungen, die zur Verwendung bei der Herstellung von Dialysatlösungen zur Verwendung in Chargen- und / oder Dosiersystemen und zur Verbesserung der Dialyseeffizienz durch Reduzieren oder Verhindern der Gerinnung in den Dialyseströmungswegen geeignet sind. Aus der EP0597817B1 ist ein Blutbehandlungsgerät bekannt, bei dem der Dialysat-Bypass-Status mithilfe der Durchflussmessung ermittelt wird. Darüberhinaus offenbart die EP0311848A2 eine Vorrichtung für die Hämodialyse mit einer Mischeinrichtung, die Dialysierflüssigkeit für einen Dialysator durch Mischung von Konzentrat mit aufbereitetem Wasser herstellt.

Aus der DE 34 16 057 A1 ist ein Dialysegerät bekannt, bei dem eine Leitfähigkeitsmesszelle stromabwärts der Einmündungen der Konzentratleitungen in eine Hauptleitung angeordnet ist. Die Leitfähigkeitsmesszelle dient zur Ermittlung der Leitfähigkeit der in den Dialysator einströmenden Dialyselösung. Damit lässt sich zwar eine Überprüfung der Gesamtleitfähigkeit vornehmen, eine Überwachung des Bicarbonatgehaltes der Dialyselösung ist in der DE 34 16 057 A1 nicht vorgesehen. Ein ähnliches Dialysegerät ist aus US 2016/051949 bekannt.

Der vorliegenden Erfindung liegt die Aufgabe zugrunde, ein Verfahren der eingangs genannten Art dahingehend weiterzubilden, dass auf möglichst einfache Art und Weise und zuverlässig eine Überwachung des Natriumgehalts und des Bicarbonat-gehalts der Dialyselösung möglich ist.

Diese Aufgabe wird durch ein Verfahren mit den Merkmalen des Anspruchs 1 gelöst.

Danach umfasst das Verfahren die folgenden Schritte, die in der angegebenen (zeitlichen) Reihenfolge, aber auch in jeder anderen möglichen Reihenfolge durchgeführt werden können.

a. Zugeben der sauren Natriumkomponente und Messung der Leitfähigkeit ($LF_{ist,Na}$),

b. Zugeben der Bicarbonat-Komponente und Messung des durch das Zugeben der Bicarbonat Komponente bedingten Anstieges der Leitfähigkeit ($\Delta LF_{ist,Bic}$),

c. Ermittlung des durch die Zugabe der Bicarbonat Komponente erwarteten Anstieges der Leitfähigkeit ($\Delta LF_{exp,Bic}$),

d. Prüfung, ob der gemessene Anstieg der Leitfähigkeit ($\Delta LF_{ist,Bic}$) in einem erwarteten Bereich des Anstieges der Leitfähigkeit ($\Delta LF_{exp,Bic}$) liegt,

e. Ermittlung der nach der Zugabe der Bicarbonat-Komponente und der sauren Natriumkomponente erwarteten Gesamtleitfähigkeit ($LF_{exp,D}$),

f. Messung der Gesamtleitfähigkeit ($LF_{ist,D}$) nach der Zugabe der Bicarbonat-Komponente und der sauren Natriumkomponente und

g. Prüfung, ob die gemessene Gesamtleitfähigkeit ($LF_{ist,D}$) in einem erwarteten Bereich der Gesamtleitfähigkeit ($LF_{exp,D}$) liegt.

wobei die Messung der Leitfähigkeit gemäß Schritt a., die Messung des Anstieges der Leitfähigkeit gemäß Schritt b. und die Messung der Gesamtleitfähigkeit gemäß Schritt f. von ein und derselben Leitfähigkeitsmesszelle durchgeführt werden.

Erfindungsgemäß wird ein erwarteter Anstieg der Leitfähigkeit durch die Zugabe der Bicarbonat-Komponente mit einem gemessenen Anstieg der Leitfähigkeit verglichen und auf diese Weise rückgeschlossen, ob die Bicarbonat-Konzentration der Dialyselösung in dem gewünschten Bereich liegt.

Die Überwachung der Natrium-Konzentration erfolgt basierend auf einem Vergleich zwischen der gemessenen Gesamtleitfähigkeit, d.h. der gemessenen Leitfähigkeit der Dialyselösung nach Zugabe der sauren Natriumkomponente sowie der Bicarbonat-Komponente. Auf der Basis dieses Vergleichs kann rückgeschlossen werden, ob die Natrium-Konzentration in dem gewünschten Bereich liegt.

Für diese Leitfähigkeitsmessungen wird ein einziger Leitfähigkeitssensor verwendet, was gegenüber bekannten Anordnungen eine Vereinfachung darstellt. Mittels des vorliegenden Verfahrens ist es möglich, mit einem einzigen Leitfähigkeitssensor sowohl die Bicarbonat-Konzentration als auch die Natrium-Konzentration zu überwachen.

Die Messgröße ist die Gesamtleitfähigkeit der Dialyselösung bzw. deren Änderung über die Zeit. Das erfindungsgemäße Verfahren kann dazu verwendet werden, die normativ geforderten Grenzen für Bicarbonat- und Natriumabweichungen einer Dialyselösung beispielsweise durch zwei unabhängige und/oder unterschiedliche Grenzwertfenster zu überwachen. Die Dialyselösung wird vorzugsweise durch das separate Zudosieren der Komponenten (saure Natriumkomponente und Bicarbonat-Komponente) erhalten.

Der Begriff "saure Natriumkomponente" umfasst jede beliebige Anordnung eines Natrium-Ionen enthaltenden Mediums, insbesondere eines Natrium-Ionen enthaltenden Konzentrats, das wenigstens eine Säure enthält. Vorzugsweise enthält die Natrium-Komponente weitere Elektrolyte, wie z.B. Ca, Mg, Cl, K und ggf. weitere Inhaltsstoffe. Vorzugsweise ist vorgesehen, dass die Natrium-Komponente hauptsächlich Natrium-Ionen enthält.

Vorzugsweise ist die saure Natriumkomponente die Hauptquelle von Natrium in der fertigen Dialyselösung, d.h. vorzugsweise stammt der überwiegende Teil des Natriums der fertigen Dialyselösung aus der sauren Natriumkomponente.

Der Begriff "Bicarbonat-Komponente" umfasst jede beliebige Anordnung eines Bicarbonat-Ionen enthaltenden Mediums, insbesondere eines Bicarbonat-Ionen enthaltenden Konzentrats. Vorzugsweise enthält die Bicarbonat-Komponente neben Bicarbonat Elektrolyte, wie z.B. Na-Ionen. Vorzugsweise ist vorgesehen, dass die Bicarbonat-Komponente hauptsächlich Bicarbonat-Ionen enthält.

Die Bicarbonat-Komponente ist vorzugsweise basisch.

In einer bevorzugten Ausgestaltung der Erfindung enthält die Bicarbonat-Komponente keine Säure.

Der im Rahmen der Erfindung verwendete Begriff "Messung" umfasst die unmittelbare Messung einer Größe, z.B. der Leitfähigkeit, sowie auch Werte, auf die Messungen basieren, wie z.B. Werte, die durch Subtraktion zweier unmittelbar gemessener Werte der Leitfähigkeit erhalten werden.

Der im Rahmen der Erfindung verwendete Begriff "Zugeben" umfasst die Beimischung einer Komponente in eine andere Komponente, wie z.B. in Wasser sowie auch die Vorlage der Komponente. Unter dem Begriff "Zugeben einer sauren Natriumkomponente" ist somit beispielsweise sowohl der Fall zu verstehen, dass die saure Natriumkomponente einer anderen Komponente, wie z.B. RO-Wasser beigefügt wird sowie auch der Fall, dass diese Komponente vorgelegt wird, d.h. z.B. eine Mischung aus einem Natrium-haltigen Konzentrat und RO-Wasser.

In einer denkbaren Ausgestaltung der Erfindung wird die saure Natriumkomponente und Wasser gemischt und an der Leitfähigkeitsmesszelle vorbeigeführt und deren Leitfähigkeit gemessen. In einem zweiten Schritt kann dann die Bicarbonat-Komponente hinzugegeben werden und es kann die Gesamtleitfähigkeit ermittelt werden. Die Messung des Anstieges der Leitfähigkeit durch die Zugabe der Bicarbonat-Komponente kann dadurch vorgenommen werden, dass von der gemessenen Gesamtleitfähigkeit die gemessene Leitfähigkeit abgezogen wird, die die Mischung aus Wasser und der sauren Natriumkomponente (vor der Zugabe der Bicarbonat-Komponente) aufweist.

Liegt der gemessene Anstieg der Leitfähigkeit außerhalb eines bestimmten Bereiches, kann darauf geschlossen werden, dass die Bicarbonat-Konzentration nicht der gewünschten Bicarbonat-Konzentration entspricht.

Liegt die gemessene Gesamtleitfähigkeit außerhalb eines bestimmten Bereiches, kann darauf geschlossen werden, dass die Natrium-Konzentration nicht der gewünschten Natrium-Konzentration entspricht. Denn die Gesamtleitfähigkeit der Dialyselösung ist hauptsächlich sensitiv gegenüber Veränderungen der Natrium-Konzentration.

Auf diese Weise kann mittels eines einzigen Leitfähigkeitssensors permanent oder intervallweise während der Herstellung der Dialyselösung sowohl der Gehalt der Bicarbonat- als auch der Gehalt an Natrium-Ionen überwacht werden.

Wie oben ausgeführt, besteht eine denkbare Variante des Verfahrens darin, dass zunächst die saure Natriumkomponente zugegeben wird und dass der gemessene Anstieg der Leitfähigkeit durch die Zugabe der Bicarbonat-Komponente gemäß Schritt b. dadurch erhalten wird, dass die gemessene Leitfähigkeit nach dem Zugeben der sauren Natriumkomponente von der gemessenen Gesamtleitfähigkeit abgezogen wird.

Der erwartete Anstieg der Leitfähigkeit gemäß Schritt c, d.h. der erwartete Anstieg der Leitfähigkeit durch die Zugabe der der Bicarbonat-Komponente kann aus den Inhaltsstoffen der Bicarbonat-Komponente berechnet werden.

Durch die vorliegende Erfindung ist es möglich, die Bicarbonat-Konzentration sowie auch die Natrium-Konzentration zu messen, wobei sich für die Prüfung, ob sich die Konzentrationen in gewünschten Bereichen bewegen, Grenzwertbereiche identischer oder unterschiedliche Größe definieren lassen.

Denkbar ist es beispielsweise, dass sich der erwartete Bereich für die Bicarbonat-Konzentration von einem Wert 25% unterhalb des erwarteten Anstiegs der Leitfähigkeit bis zu einem Wert 25% oberhalb des erwarteten Anstiegs der Leitfähigkeit erstreckt. Der Sollwertbereich für Bicarbonat erstreckt sich somit ausgehend von dem erwarteten Wert je 25 % nach oben und nach unten.

Für die Natrium-Konzentration kann der erwartete Bereich beispielsweise von 5 % unterhalb bis 5 % oberhalb eines erwarteten Wertes für die Gesamtleitfähigkeit erstrecken.

Grundsätzlich sind auch andere Bereichsgrenzen denkbar und von der Erfindung mit umfasst.

Vorzugsweise ist vorgesehen, dass die erwartete Gesamtleitfähigkeit aus den Inhaltsstoffen der sauren Natriumkomponente und der Bicarbonat-Komponente berechnet wird.

Um Veränderungen der Konzentrationen vornehmen zu können, während ein Patient an das Dialysegerät angeschlossen ist, kann vorgesehen sein, dass zur Überwachung des Bicarbonat-Gehalts sowie des Natrium-Gehalts einer Dialyselösung bei einem Konzentrationswechsel die hergestellte Dialyselösung zeitweise von dem Dialysator des Dialysegerätes entkoppelt wird. So kann ein "Einmessen" der Dialyselösung stattfinden, ohne dass eine Beeinflussung des Patienten erfolgt.

Bei Über- oder Unterschreiten des jeweiligen Bereiches kann ein Alarm und/oder eine Ausgabe einer entsprechenden Information an einen Nutzer ausgegeben werden, dass die gewünschte Konzentration außerhalb des Sollwertbereiches liegt.

Dabei ist es denkbar, dass ein Alarm ausgelöst wird und/oder die Ausgabe einer entsprechenden Information erfolgt, sobald der Bereich für den Natrium-Gehalt oder der Bereich für den Bicarbonat-Gehalt über- oder unterschritten ist, je nachdem welche Über- oder Unterschreitung früher eintritt. Somit wird das Überwachungsfenster, das zuerst verlassen wird, einen Alarm oder eine sonstige Information ausgeben.

Die vorliegende Erfindung betrifft des Weiteren ein Dialysegerät und/oder eine Zubereitungseinrichtung für eine Dialyselösung, das/die Mittel aufweist, die geeignet und bestimmt sind, das erfindungsgemäße Verfahren auszuführen.

Die Zubereitungseinrichtung zur Herstellung einer Dialyselösung aus wenigstens zwei Komponenten umfasst einen Behälter mit einer Bicarbonat-Komponente (Bicarbonat-Behälter") und einen Behälter mit einer sauren Natriumkomponente ("Natrium-Behälter"), wobei die Zubereitungseinrichtung eine beispielsweise stromabwärts des Natrium-Behälters angeordnete Leitfähigkeitsmesszelle zur Messung der Leitfähigkeit der Flüssigkeit nach dem Zugeben der sauren Natriumkomponente, eine vorzugsweise stromabwärts des Bicarbonat-Behälters angeordnete Leitfähigkeitsmesszelle zur Messung des durch das Zugeben der Bicarbonat-Komponente bedingten Anstieges der Leitfähigkeit und eine vorzugsweise stromabwärts des Natrium-Behälters und des Bicarbonat-Behälters angeordnete Leitfähigkeitsmesszelle zur Messung der Gesamtleitfähigkeit der die saure Natriumkomponente und die Bicarbonat-Komponente enthaltenden Dialyselösung aufweist. Des Weiteren weist das Dialysegerät eine Prüfeinheit auf, die derart ausgebildet ist, dass diese prüft, ob der durch die Zugabe der Bicarbonat-Komponente bedingte gemessene Anstieg der Leitfähigkeit einem erwarten Bereich des Anstiegs der Leitfähigkeit liegt und ob die gemessene Gesamtleitfähigkeit in einem erwarteten Bereich der Gesamtleitfähigkeit liegt, wobei es sich bei den genannten Leitfähigkeitsmesszellen um ein und dieselbe Leitfähigkeitsmesszelle handelt.

Vorzugsweise weist die Zubereitungseinrichtung eine Alarmeinheit und/oder Ausgabeeinheit zur Ausgabe einer Information an einen Nutzer auf, die derart ausgebildet sind, dass ein Alarm ausgelöst bzw. eine Information ausgegeben wird, wenn die Über- oder Unterschreitung des Bereiches erfasst wird.

Die Zubereitungseinrichtung kann eine Hauptleitung aufweisen, in die eine mit dem Bicarbonat-Behälter in Verbindung stehende Zuleitung mündet und die eine weitere mit dem Natrium-Behälter in Verbindung stehende Zuleitung mündet. Diese Hauptleitung kann mit einem Behälter oder mit einer Quelle für Wasser, vorzugsweise für RO-Wasser in Verbindung stehen.

In das durch die Hauptleitung strömende Fluid, insbesondere RO-Wasser, wird somit die Bicarbonat-Komponente sowie die saure Natriumkomponente zudosiert. Dazu können Pumpen eingesetzt werden, die die Komponenten aus Konzentratbehältern in die Hauptleitung fördern.

Auch ist es denkbar und von der Erfindung mit umfasst, dass die die Komponenten enthaltenden Behälter durchströmt werden. So kann die Herstellung der Dialyselösung beispielsweise so erfolgen, dass RO-Wasser oder eine sonstige zur Herstellung vorgesehene Flüssigkeit den Behälter enthaltend das Bicarbonat oder enthaltend das Natrium durchströmt und so eine Bicarbonat- bzw. Natrium-haltige Lösung hergestellt wird. In diese Lösung kann sodann die andere Komponente zudosiert werden, was beispielsweise durch Einleiten eines Konzentrats in eine Hauptleitung oder durch Durchströmung des Konzentratbehälters erfolgen kann.

Die Leitfähigkeitsmesszelle ist vorzugsweise stromabwärts beider Einmündungen der Zuleitungen angeordnet bzw. derart, dass sowohl die Gesamtleitfähigkeit als auch der durch die Zugabe des Bicarbonats hervorgerufene Anstieg der Leitfähigkeit gemessen werden kann.

Die Zubereitungseinrichtung kann eine Bypassleitung aufweisen, die relativ zu einem Dialysator eines Dialysegerätes herumführt und durch die die hergestellte Dialyselösung zeitweise geführt wird. Wie oben ausgeführt, kann auf diese Weise sichergestellt werden, dass die fertige Dialyselösung erst dann zur Behandlung verwendet wird, wenn sie korrekt eingemessen ist und die Grenzwertfenster gesetzt worden sind, d.h. wenn sowohl der Bicarbonat-Gehalt als auch der Natrium-Gehalt in den jeweils gewünschten Bereichen liegen.

Die Zubereitungseinrichtung kann einen integralen Bestandteil eines Dialysegerätes aufweisen oder auch als von einem Dialysegerät separate Einheit ausgebildet sein.

Die vorliegende Erfindung betrifft des Weiteren ein Dialysegerät mit wenigstens einer Zubereitungseinrichtung gemäß der Erfindung.

Weitere Einzelheiten und Vorteile der Erfindung werden anhand eines in der Zeichnung dargestellten Ausführungsbeispiels näher erläutert.

Die Figur zeigt eine schematische Darstellung der Natrium- und Bicarbonat-Überwachung mittels zweier unabhängiger Grenzwertfenster (Bereiche) unter Verwendung einer einzigen Leitfähigkeitsmesszelle.

Das im Folgenden beschriebene Ausführungsbeispiel stellt eine Möglichkeit dar, durch eine Leitfähigkeitsmessung der Dialyselösung in einem Zweikomponentensystem zusätzlich auf die geforderten Grenzen hin zu überwachen, ohne dass dazu mehr als eine Leitfähigkeitsmesszelle verwendet werden muss. An dieser Stelle wird darauf hingewiesen, dass die Erfindung nicht auf die Zugabe von genau zwei Komponenten beschränkt ist, auch mehr als zwei Komponenten sind zugebbar.

Gemäß der Erfindung sind eine Bicarbonat-Komponente und eine saure Natriumkomponente separat zudosierbar. Das Zudosieren kann beispielsweise in eine Leitung erfolgen, in der Wasser, vorzugsweise RO-Wasser bzw. eine Lösung strömt, die aus einer Mischung einer der Komponenten mit Wasser bzw. RO-Wasser erhalten wurde.

Die erwartete Gesamtleitfähigkeit sowie der erwartete Anstieg der Leitfähigkeit nach Zugabe der Bicarbonat-Komponente sind aufgrund der jeweiligen Inhaltsstoffe bzw. aufgrund der theoretisch zu erwartenden Leitfähigkeit der Dialyselösung zu berechnen.

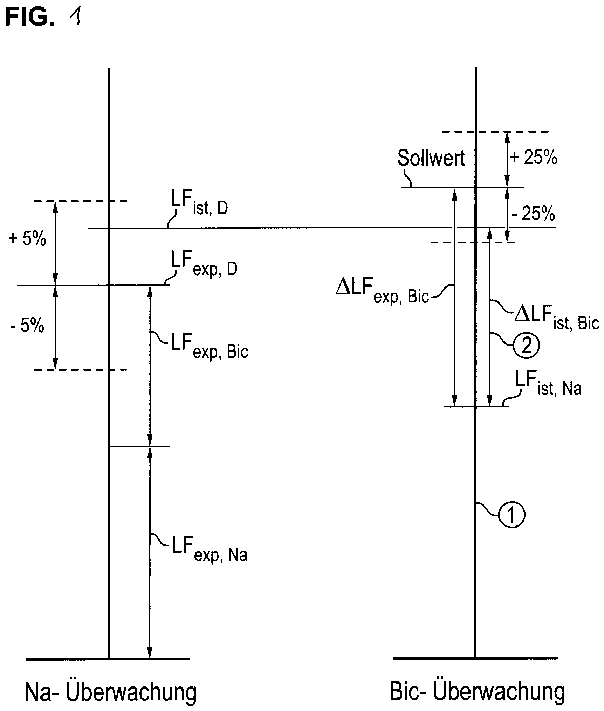

Gemäß Figur 1 wird mit Hilfe eines mathematischen Formalismus die zu erwartende Leitfähigkeit, d.h. die zu erwartende Gesamtleitfähigkeit $LF_{exp,D}$ der fertigen Dialyselösung aus deren Inhaltsstoffen berechnet.

Das Betriebssystem, d.h. die Zubereitungseinrichtung, in dem die Dialyselösung gemischt wird, ist mit verschiedenen Toleranzen behaftet. Diese Toleranzkette kann dazu führen, dass die tatsächlich gemessene Gesamtleitfähigkeit $LF_{ist,D}$ der fertigen, d.h. die saure Natriumkomponente und die Bicarbonat-Komponente enthaltende Dialyselösung von dem theoretisch berechneten Erwartungswert $LF_{exp,D}$ abweicht.

Wie dies aus Figur 1, linke Seite ("Na-Überwachung") hervorgeht, setzt sich die zu erwartende Gesamtleitfähigkeit $LF_{exp,D}$ aus der erwarteten (nominalen) Leitfähigkeit der sauren Natriumkomponente $LF_{exp,Na}$ sowie aus der erwarteten (nominalen) Leitfähigkeit der Bicarbonat-Komponente $LF_{exp,Bic}$ zusammen.

Durch Überwachung der Gesamtleitfähigkeit $LF_{exp,D}$ auf eine in Figur 1, linke Seite gezeigte Abweichung von 5 %, kann eine 5 %-ige Abweichung der Natriumkonzentration gegenüber dem berechneten Erwartungswert $LF_{exp,D}$, d.h. vom Sollwert ermittelt werden. Der Bereich, in dem die Gesamtleitfähigkeit liegen darf, beträgt somit 10 % und erstreckt sich von dem Erwartungswert je 5 % nach oben und nach unten.

Die in Figur 1, rechte Seite ("Bic-Überwachung") gestaltet sich wie folgt:

Die Leitfähigkeit $LF_{ist,Na}$ ist die gemessene Leitfähigkeit der Lösung nach dem Zugeben der sauren Natriumkomponente.

Der erwartete Anstieg der Leitfähigkeit durch Zugabe der Bicarbonat-Komponente zu dieser Lösung ist in Figur 1, rechte Seite mit $\Delta LF_{exp,Bic}$ bezeichnet. Er berechnet sich aus der Differenz zwischen der erwarteten Gesamtleitfähigkeit $LF_{exp,D}$ und der erwarteten Leitfähigkeit der sauren Natriumkomponente $LF_{exp,Na}$.

Neben Bicarbonat enthält die Bicarbonat-Komponente üblicherweise Natrium. Um den Einfluss der sauren Natrium-haltigen Komponente selbst, die toleranzbehaftete Dosierung der sauren Natriumkomponente sowie zusätzliche Störgrößen, wie z.B. die Grundleitfähigkeit des verwendeten Wassers bei der Berechnung des Sollwertes der Leitfähigkeit der Bicarbonat-Komponente nicht berücksichtigen zu müssen, wird wie folgt vorgegangen:

In einem ersten Schritt wird lediglich die saure Natriumkomponente und das notwendige Wasser zudosiert (Dosierung 1) und an der Leitfähigkeitsmesszelle vorbeigeführt. Die dabei erhaltene Leitfähigkeit $LF_{ist,Na}$ beinhaltet sämtliche Toleranzen des Betriebssystems und dient für die noch zu bestimmenden Überwachungsgrenzen des Bicarbonats als Offset.

Im zweiten Schritt wird die Bicarbonat-Komponente hinzugegeben (Dosierung 2), so dass sämtliche Komponenten vorhanden sind und die Dialyselösung vollständig angemischt ist. Es stellt sich eine gemessene Gesamtleitfähigkeit $LF_{ist,D}$ der Dialyselösung ein. Die bereits bestimmte Leitfähigkeit der Natrium-haltigen Dialyselösung (ohne Bicarbonat) $LF_{ist,Na}$ wird von der gemessenen Gesamtleitfähigkeit $LF_{ist,D}$ abgezogen und so der tatsächliche Beitrag der Bicarbonat Komponente $\Delta LF_{ist,Bic}$ zur Gesamtleitfähigkeit gemessen:

$$\Delta LF_{ist,Bic} = LF_{ist,D} - LF_{ist,Na}$$

Der in Schritt 1 bestimmte Offset der sauren Natriumkomponente (inklusive Wasser und Toleranzen) wird somit aus der gemessenen Gesamtleitfähigkeit $LF_{ist,D}$ rechnerisch eliminiert und auf diese Weise der Beitrag der Bicarbonat-Komponente zur Leitfähigkeit bestimmt.

Die Überwachungsgrenzen können aus dem theoretisch zu erwartenden Anstieg der Leitfähigkeit durch die Zugabe der Bicarbonat-Komponente $\Delta LF_{exp,Bic}$ (Sollwert) berechnet werden und beziehen sich somit auf den geforderten Sollwert. In dem Ausführungsbeispiel gemäß Figur 1, rechte Seite erstrecken sich die Überwachungsgrenzen von einem Wert 25 % unter $\Delta LF_{exp,Bic}$ bis 25 % über $\Delta LF_{exp,Bic}$, d.h. der zulässige Anstieg der durch die Zugabe der Bicarbonat Lösung bedingten Leitfähigkeit liegt im Intervall $\Delta LF_{exp,Bic} * 0{,}75$ bis $\Delta LF_{exp,Bic} * 1{,}25$.

Unter Berücksichtigung der Grenzwerte für die Gesamtleitfähigkeit ergeben sich somit zwei unabhängige Überwachungsfenster für Natrium und Bicarbonat, die entsprechend der normativen Forderungen die jeweiligen Konzentrationsabweichungen von Sollwert detektieren können.

Um eine Zweikanaligkeit zu gewährleisten, ist vorzugsweise vorgesehen, dass sämtliche theoretischen Berechnungen von Erwartungswerten von einem Schutzsystem vorgenommen werden, das von dem Betriebssystem des Dialysegerätes unabhängig ist.

Veränderungen der Konzentrationen während der Behandlung, wie z.B. die Verstellung der Natrium- und/oder Bicarbonat-Konzentration können mit der erfindungsgemäßen Vorgehensweise berücksichtigt werden. Ein erneutes Einmessen des Systems, d.h. die erneute Herstellung einer Dialyselösung mit entsprechend geänderten Konzentrationswerten könnte beispielsweise während eines Bypass, d.h. während einer hydraulische Entkopplung der Zubereitungseinrichtung von Wasserteil des Dialysegerätes erfolgen. Unter Umständen wäre auch ohne ein erneutes Einmessen bei einem Wechsel der Konzentration eine theoretische Neuberechnung der Überwachungsfenster denkbar.

Bei einem Konzentrat- oder Kanisterwechsel ist das Verfahren nach einer ersten Variante erneut durchzuführen. Für den Fall, dass eine etwas größere Unsicherheit akzeptiert werden kann, ist bei einem Konzentrat- oder Kanisterwechsel nach einer zweiten Variante eine theoretische Neuberechnung des Bicarbonat-Grenzfensters möglich. Das Natrium-Grenzfenster kann jederzeit neu berechnet werden. Darüber hinaus kann die Natrium-Konzentration immer neu gemessen werden.

Durch die gleichzeitige Überwachung der Natrium- und der Bicarbonat-Konzentration mittels einer einzigen Leitfähigkeitsmesszelle kann ein plötzlich auftretender Fehler in der Zudosierung der sauren Natriumkomponente dazu führen, dass ein Alarm ausgelöst wird, weil das Bicarbonat-Überwachungsfenster verlassen wurde. Aufgrund der Tatsache, dass eine einzelne Leitfähigkeitsmesszelle, die im Normalbetrieb die Gesamtleitfähigkeit der Dialyselösung misst, keine Unterscheidung treffen kann, von welcher Komponente der Fehler herrührt, wird das Überwachungsfenster, das zuerst verlassen wird, einen Alarm auslösen. Mit Hilfe eines Risikomanagements kann ausgeschlossen werden, dass beispielsweise Zudosierfehler der die Komponenten fördernden Pumpen gegenläufig auftreten können. Dies könnte unter Umständen zu einer gegenseitigen Aufhebung der Fehler in der Leitfähigkeit führen. Mehrfachfehler werden aus Sicht des Risikomanagements ausgeschlossen.

Es wird darauf hingewiesen, dass der Begriff "Leitfähigkeit" jede Größe mit umfasst, die mit der Leitfähigkeit bzw. mit dem Gehalt von Natrium und Bicarbonat korreliert. Dementsprechend umfasst auch der Begriff "Leitfähigkeitsmesszelle" jeden Sensor, mittels dessen die Leitfähigkeit oder eine damit bzw. mit dem Gehalt von Natrium und Bicarbonat korrelierte Größe gemessen werden kann.

Für das Verfahren spielt es keine Rolle, in welcher lokalen Reihenfolge die saure Natriumkomponente und die Bicarbonat-Komponente während des normalen Betriebs in der Hydraulik des Dialysegerätes zugegeben werden.

Grundsätzlich wird darauf hingewiesen, dass alle oder ein Teil der Schritte des erfindungsgemäßen Verfahrens kontinuierlich und/oder mehrfach und/oder zyklisch und/oder periodisch durchgeführt werden können.

**Patentansprüche**

1. Verfahren zur Überwachung des Bicarbonat-Gehalts sowie des Natrium-Gehalts einer Dialyselösung, wobei die Dialyselösung unter Zugabe einer Bicarbonat-Komponente sowie einer sauren Natriumkomponente herstellt wird und wobei das Verfahren die folgenden Schritte aufweist:

   a. Zugeben der sauren Natriumkomponente und Messung der Leitfähigkeit ($LF_{ist,Na}$),
   b. Zugeben der Bicarbonat-Komponente und Messung des durch das Zugeben der Bicarbonat Komponente bedingten Anstieges der Leitfähigkeit ($\Delta LF_{ist,Bic}$),
   c. Ermittlung des durch die Zugabe der Bicarbonat Komponente erwarteten Anstieges der Leitfähigkeit ($\Delta LF_{exp,Bic}$),
   d. Prüfung, ob der gemessene Anstieg der Leitfähigkeit ($\Delta LF_{ist,Bic}$) in einem erwarteten Bereich des Anstieges der Leitfähigkeit ($\Delta LF_{exp,Bic}$) liegt,
   e. Ermittlung der nach der Zugabe der Bicarbonat-Komponente und der sauren Natriumkomponente erwarteten Gesamtleitfähigkeit ($LF_{exp,D}$),
   f. Messung der Gesamtleitfähigkeit ($LF_{ist,D}$) nach der Zugabe der Bicarbonat-Komponente und der sauren Natriumkomponente und
   g. Prüfung, ob die gemessene Gesamtleitfähigkeit ($LF_{ist,D}$) in einem erwarteten Bereich der Gesamtleitfähigkeit ($LF_{exp,D}$) liegt.

wobei die Messung der Leitfähigkeit gemäß Schritt a., die Messung des Anstieges der Leitfähigkeit gemäß Schritt b. und die Messung der Gesamtleitfähigkeit gemäß Schritt f. von ein und derselben Leitfähigkeitsmesszelle durchgeführt werden.

2. Verfahren nach Anspruch 1, **dadurch gekennzeichnet, dass** zunächst die saure Natriumkomponente zugegeben wird und dass die Messung des Anstiegs der Leitfähigkeit ($\Delta LF_{ist,Bic}$) gemäß Schritt b. dadurch erhalten wird, dass die gemessene Leitfähigkeit ($LF_{ist,Na}$) nach dem Zugeben der sauren Natriumkomponente von der gemessenen Gesamtleitfähigkeit ($LF_{ist,D}$) gemäß Schritt f. abgezogen wird.

3. Verfahren nach Anspruch 1 oder 2, **dadurch gekennzeichnet, dass** der erwartete Anstieg der Leitfähigkeit ($\Delta LF_{exp,Bic}$) gemäß Schritt c. aus den Inhaltsstoffen der Bicarbonat-Komponente berechnet wird.

4. Verfahren nach einem der vorhergehenden Ansprüche, **dadurch gekennzeichnet, dass** sich der erwartete Bereich gemäß Schritt d. von einem Wert 25% unterhalb des erwarteten Anstiegs der Leitfähigkeit ($\Delta LF_{exp,Bic}$) bis zu einem Wert 25% oberhalb des erwarteten Anstiegs der Leitfähigkeit ($\Delta LF_{exp,Bic}$) erstreckt.

5. Verfahren nach einem der vorhergehenden Ansprüche, **dadurch gekennzeichnet, dass** die erwartete Gesamtleitfähigkeit ($\Delta LF_{exp,D}$) gemäß Schritt e. aus den Inhaltsstoffen der sauren Natriumkomponente und der Bicarbonat-Komponente berechnet wird.

6. Verfahren nach einem der vorhergehenden Ansprüche, **dadurch gekennzeichnet, dass** sich der erwartete Bereich für die Gesamtleitfähigkeit von einem Wert 5% unterhalb der erwarteten Gesamtleitfähigkeit ($\Delta LF_{exp,D}$) bis zu einem Wert 5% oberhalb der erwarteten Gesamtleitfähigkeit ($\Delta LF_{exp,D}$) erstreckt.

7. Verfahren nach einem der vorhergehenden Ansprüche, **dadurch gekennzeichnet, dass** zur Überwachung des Bicarbonat-Gehalts sowie des Natrium-Gehalts einer Dialyselösung bei einem Konzentrationswechsel die hergestellte Dialyselösung zeitweise von dem Dialysator eines Dialysegerätes entkoppelt wird.

8. Verfahren nach einem der vorhergehenden Ansprüche, **dadurch gekennzeichnet, dass** bei Über- oder Unterschreiten des Bereiches ein Alarm und/oder eine Ausgabe einer entsprechenden Information an einen Nutzer generiert wird.

9. Verfahren nach Anspruch 8, **dadurch gekennzeichnet, dass** ein Alarm ausgelöst wird und/oder die Ausgabe einer entsprechenden Information erfolgt, sobald der Bereich für den Natrium-Gehalt oder der Bereich für den Bicarbonat-Gehalt über- oder unterschritten ist, je nachdem welche Über- oder Unterschreitung früher eintritt.

10. Zubereitungseinrichtung zur Herstellung einer Dialyselösung aus wenigstens zwei Komponenten, wobei die Zubereitungseinrichtung dazu ausgelegt ist, ein Verfahren nach einem der Ansprüche 1 bis 9 auszuführen und einen Behälter mit einer Bicarbonat-Komponente (Bicarbonat-Behälter") und einen Behälter mit einer sauren Natriumkomponente ("Natrium-Behälter") aufweist, wobei die Zubereitungseinrichtung eine vorzugsweise stromabwärts des Natrium-Behälters angeordnete Leitfähigkeitsmesszelle, welche dazu ausgelegt ist, die Leitfähigkeit ($LF_{ist,Na}$) der Flüssigkeit nach dem Zugeben der sauren Natriumkomponente zu messen, eine vorzugsweise stromabwärts des Bicarbonat-Behälters angeordnete Leitfähigkeitsmesszelle, welche dazu ausgelegt ist, dendurch das Zugeben der Bicarbonat-haltigen Komponente bedingten Anstieg der Leitfähigkeit ($\Delta LF_{ist,Bic}$) zu messen, und eine vorzugsweise stromabwärts des Natrium-Behälters und des Bicarbonat-Behälters angeordnete Leitfähigkeitsmesszelle aufweist, welche dazu ausgelegt ist, die Gesamtleitfähigkeit ($LF_{ist,D}$) der die saure Natriumkomponente und die Bicarbonat-Komponente enthaltenden Dialyselösung zu messen, und wobei die Zubereitungseinrichtung eine Prüfeinheit aufweist, die derart ausgebildet ist, dass diese prüft, ob der durch die Zugabe der Bicarbonat-Komponente bedingte gemessene Anstieg der Leitfähigkeit ($\Delta LF_{ist,Bic}$) in einem erwarten Bereich des Anstiegs der Leitfähigkeit ($\Delta LF_{exp,Bic}$) liegt und ob die gemessene Gesamtleitfähigkeit ($LF_{ist,D}$) in einem erwarteten Bereich der Gesamtleitfähigkeit ($LF_{exp,D}$) liegt, wobei es sich bei den genannten Leitfähigkeitsmesszellen um ein und dieselbe Leitfähigkeitsmesszelle handelt.

11. Zubereitungseinrichtung nach Anspruch 10, **dadurch gekennzeichnet, dass** die Zubereitungseinrichtung eine Alarmeinheit und/oder Ausgabeeinheit zur Ausgabe einer Information an einen Nutzer aufweist, die derart ausgebildet sind, dass ein Alarm ausgelöst bzw. eine Information ausgegeben wird, wenn die Über- oder Unterschreitung eines Bereiches erfasst wird.

12. Zubereitungseinrichtung nach Anspruch 10 oder 11, **dadurch gekennzeichnet, dass** die Zubereitungseinrichtung eine Hauptleitung aufweist, in die eine mit dem Bicarbonat-Behälter in Verbindung stehende Zuleitung mündet und in die eine weitere mit dem Natrium-Behälter in Verbindung stehende Zuleitung mündet und/oder dass die Hauptleitung mit einem Behälter oder mit einer Quelle für Wasser, vorzugsweise für RO-Wasser in Verbindung steht und/oder dass die Leitfähigkeitsmesszelle stromabwärts beider Einmündungen der Zuleitungen angeordnet ist.

13. Zubereitungseinrichtung nach einem der Ansprüche 10 bis 12, **dadurch gekennzeichnet, dass** die Zubereitungseinrichtung mit einem Dialysator eines Dialysegerätes in Fluidverbindung gebracht werden kann, so dass die hergestellte Dialyselösung die Dialysatseite des Dialysators durchströmt und dass die Zubereitungseinrichtung eine Bypassleitung aufweist, die um den Dialysator herumführt, so dass die hergestellte Dialyselösung zeitweise um den Dialysator herumgeführt werden kann.

14. Dialysegerät mit einer Zubereitungseinrichtung gemäß einem der Ansprüche 10 bis 13.

**Claims**

1. A method of monitoring the bicarbonate content and the sodium content of a dialysis solution, wherein the dialysis solution is prepared while adding a bicarbonate component and an acidic sodium component, and wherein the method comprises the following steps:

   a. adding the acidic sodium component and measuring the conductivity ($LF_{ist,Na}$);
   b. adding the bicarbonate component and measuring the increase in conductivity ($\Delta LF_{ist,Bic}$) caused by adding the bicarbonate component;
   c. determining the increase in conductivity ($\Delta LF_{exp,Bic}$) expected due to the addition of the bicarbonate component;
   d. checking whether the measured increase in conductivity ($\Delta LF_{ist,Bic}$) lies in an expected range of the increase in conductivity ($\Delta LF_{exp,Bic}$);
   e. determining the total conductivity ($LF_{exp,D}$) expected after the addition of the bicarbonate component and of the acidic sodium component;
   f. measuring the total conductivity ($LF_{ist,D}$) after the addition of the bicarbonate component and of the acidic sodium component; and
   g. checking whether the measured total conductivity $LF_{ist,D}$) lies in an expected range of the total conductivity ($LF_{exp,D}$),

   wherein the measurement of the conductivity in accordance with step a.; the measurement of the increase in conductivity in accordance with step b.;

and the measurement of the total conductivity in accordance with step f. are carried out by one and the same conductivity measurement cell.

2. A method in accordance with claim 1, **characterized in that** the acidic sodium component is added first; and **in that** the measurement of the increase in conductivity ($\Delta LF_{ist,Bic}$) in accordance with step b. is obtained **in that** the measured conductivity ($LF_{ist,Na}$) after the addition of the acidic sodium component is deducted from the measured total conductivity ($LF_{ist,D}$) in accordance with step f.

3. A method in accordance with claim 1 or claim 2, **characterized in that** the expected increase in conductivity ($\Delta LF_{exp,Bic}$) in accordance with step c. is calculated from the substances of the bicarbonate component.

4. A method in accordance with one of the preceding claims, **characterized in that** the expected range in accordance with step d. extends from a value 25% below the expected increase in conductivity ($\Delta LF_{exp,Bic}$) up to a value 25% above the expected increase in conductivity ($\Delta LF_{exp,Bic}$).

5. A method in accordance with one of the preceding claims, **characterized in that** the expected total conductivity ($\Delta LF_{exp,D}$) in accordance with step e. is calculated from the substances of the acidic sodium component and of the bicarbonate component.

6. A method in accordance with one of the preceding claims, **characterized in that** the expected range for the total conductivity extends from a value 5% below the expected total conductivity ($\Delta LF_{exp,D}$) up to a value 5% above the expected total conductivity ($\Delta LF_{exp,D}$).

7. A method in accordance with one of the preceding claims, **characterized in that** the prepared dialysis solution is decoupled from the dialyzer of a dialysis machine from time to time for monitoring the bicarbonate content and the sodium content of a dialysis solution on a change in concentration.

8. A method in accordance with one of the preceding claims, **characterized in that** an alarm and/or an output of corresponding information to a user is/are generated on an exceeding or a falling below of the range.

9. A method in accordance with claim 8, **characterized in that** an alarm is triggered and/or the output of corresponding information takes place as soon as the range for the sodium content or the range for the bicarbonate content is exceeded or fallen below depending on which exceeding or falling below takes place the sooner.

10. A preparation device for preparing a dialysis solution from at least two components, wherein the preparation device is configured to carry out a method in accordance with one of the claims 1 to 9 and has a container having a bicarbonate component ("bicarbonate container") and a container having an acidic sodium component ("sodium container"); wherein the preparation device has a conductivity measurement cell, preferably arranged downstream of the sodium container which is configured to measure the conductivity ($LF_{ist,Na}$) of the fluid after the addition of the acidic sodium component; a conductivity measurement cell, preferably arranged downstream of the bicarbonate container which is configured to measure the increase in conductivity ($\Delta LF_{ist,Bic}$) caused by the addition of the component containing bicarbonate; and a conductivity measurement cell, preferably arranged downstream of the sodium container and of the bicarbonate container which is configured to measure the total conductivity ($LF_{ist,D}$) of the dialysis solution containing the acidic sodium component and the bicarbonate component; and wherein the preparation device has a checking unit which is configured such that it checks whether the increase in conductivity ($\Delta LF_{ist,Bic}$) caused by the addition of the bicarbonate component is in an expected range of the increase in conductivity ($\Delta LF_{exp,Bic}$) and whether the measured total conductivity ($LF_{ist,D}$) is in an expected range of the total conductivity ($LF_{exp,D}$), with the named conductivity measurement cells being one and the same conductivity measurement cell.

11. A preparation device in accordance with claim 10, **characterized in that** the preparation device has an alarm unit and/or an output unit for outputting information to a user which is/are configured such that an alarm is triggered and/or information is output when the exceeding or falling below of the range is detected.

12. A preparation device in accordance with claim 10 or claim 11, **characterized in that** the preparation device has a main line into which a feed line in communication with the bicarbonate container opens and into which a further feed line in communication with the sodium container opens; and/or **in that** the main line is in communication with a container or with a source for water, preferably for RO water; and/or **in that** the conductivity measurement cell is arranged downstream of both openings of the feed lines.

13. A preparation device in accordance with one of the claims 10 to 12, **characterized in that** the preparation device can be brought into fluid communication with a dialyzer of a dialysis machine so that the prepared dialysis solution flows through the dialyzate side of the dialyzer; and **in that** the preparation device has a bypass line which leads around the dialyzer so that the prepared dialysis solution can be led around the dialyzer at times.

14. A dialysis machine having a preparation device in accordance with one of the claims 10 to 13.

## Revendications

1. Procédé pour surveiller la teneur en bicarbonate ainsi que la teneur en sodium d'un dialysat, le dialysat étant obtenu en ajoutant un composant bicarbonate ainsi qu'un composant sodium acide et le procédé comportant les étapes suivantes consistant à :

   a. ajouter le composant sodium acide et mesurer la conductivité ($LF_{ist,Na}$),
   b. ajouter le composant bicarbonate et mesurer la hausse de la conductivité ($\Delta LF_{ist,Bic}$) provoquée par l'ajout du composant bicarbonate,
   c. déterminer la hausse prévue de la conductivité ($\Delta LF_{exp,Bic}$) provoquée par l'ajout du composant bicarbonate,
   d. vérifier si la hausse mesurée de la conductivité ($\Delta LF_{ist,Bic}$) se situe dans une plage prévue de la hausse de la conductivité ($\Delta LF_{exp,Bic}$),
   e. déterminer la conductivité totale prévue ($LF_{exp,D}$) après l'ajout du composant bicarbonate et du composant sodium acide,
   f. mesurer la conductivité totale ($LF_{ist,D}$) après l'ajout du composant bicarbonate et du composant sodium acide et
   g. vérifier si la conductivité totale ($LF_{ist,D}$) mesurée se situe dans une plage prévue de la conductivité totale ($LF_{exp,D}$),

   la mesure de la conductivité selon l'étape a., la mesure de la hausse de la conductivité selon l'étape b. et la mesure de la conductivité totale selon l'étape f. étant effectuées par une seule et même cellule de mesure de conductivité.

2. Procédé selon la revendication 1, **caractérisé en ce que** le composant sodium acide est d'abord ajouté et **en ce que** la mesure de la hausse de conductivité ($\Delta LF_{ist,Bic}$) selon l'étape b. est obtenue en soustrayant la conductivité ($LF_{ist,Na}$) mesurée après l'ajout du composant sodium acide de la conductivité totale ($LF_{ist,D}$) mesurée selon l'étape f..

3. Procédé selon la revendication 1 ou 2, **caractérisé en ce que** la hausse prévue de la conductivité ($\Delta LF_{exp,Bic}$) selon l'étape c. est calculée à partir des constituants du composant bicarbonate.

**4.** Procédé selon l'une des revendications précédentes, **caractérisé en ce que** la plage prévue selon l'étape d. s'étend d'une valeur 25 % inférieure à la hausse prévue de la conductivité ($\Delta LF_{exp,Bic}$) à une valeur 25 % supérieure à la hausse prévue de la conductivité ($\Delta LF_{exp,Bic}$).

**5.** Procédé selon l'une des revendications précédentes, **caractérisé en ce que** la conductivité totale prévue ($\Delta LF_{exp,D}$) selon l'étape e. est calculée à partir des constituants du composant sodium acide et du composant bicarbonate.

**6.** Procédé selon l'une des revendications précédentes, **caractérisé en ce que** la plage prévue pour la conductivité totale s'étend d'une valeur 5 % inférieure à la conductivité totale prévue ($\Delta LF_{exp,D}$) à une valeur 5 % supérieure à la conductivité totale prévue ($\Delta LF_{exp,D}$).

**7.** Procédé selon l'une des revendications précédentes, **caractérisé en ce que**, pour surveiller la teneur en bicarbonate ainsi que la teneur en sodium d'un dialysat, lors d'un changement de concentration, le dialysat obtenu est temporairement découplé du dialyseur d'un appareil de dialyse.

**8.** Procédé selon l'une des revendications précédentes, **caractérisé en ce qu'**une alarme et/ou une sortie d'une information correspondante est générée pour un utilisateur lors d'un dépassement par le haut ou par le bas de la plage.

**9.** Procédé selon la revendication 8, **caractérisé en ce qu'**une alarme est déclenchée et/ou la sortie d'une information correspondante est effectuée dès que les valeurs deviennent supérieures ou inférieures à la plage pour la teneur en sodium ou la plage pour la teneur en bicarbonate, selon quel dépassement par le haut ou par le bas se produit plus tôt.

**10.** Dispositif de préparation pour l'obtention d'un dialysat à partir d'au moins deux composants, le dispositif de préparation étant conçu pour exécuter un procédé selon l'une des revendications 1 à 9 et comportant un récipient avec un composant bicarbonate (« récipient de bicarbonate ») et un récipient avec un composant sodium acide (« récipient de sodium »), le dispositif de préparation comportant une cellule de mesure de conductivité disposée de préférence en aval du récipient de sodium, qui est conçue pour mesurer la conductivité ($LF_{ist,Na}$) du liquide après l'ajout du composant sodium acide, une cellule de mesure de conductivité disposée de préférence en aval du récipient de bicarbonate, qui est conçue pour mesurer la hausse de la conductivité ($\Delta LF_{ist,Bic}$) provoquée par l'ajout du composant contenant du bicarbonate, et une cellule de mesure de conductivité disposée de préférence en aval du récipient de sodium et du récipient de bicarbonate, qui est conçue pour mesurer la conductivité totale ($LF_{ist,D}$) du dialysat contenant le composant sodium acide et le composant bicarbonate, et le dispositif de préparation comportant une unité de vérification, qui est réalisée de manière à vérifier si la hausse de la conductivité ($\Delta LF_{ist,Bic}$) mesurée provoquée par l'ajout du composant bicarbonate se situe dans une plage prévue de la hausse de la conductivité ($\Delta LF_{exp,Bic}$) et si la conductivité totale ($LF_{ist,D}$) mesurée se situe dans une plage prévue de la conductivité totale ($LF_{exp,D}$), lesdites cellules de mesure de conductivité étant une seule et même cellule de mesure de conductivité.

**11.** Dispositif de préparation selon la revendication 10, **caractérisé en ce que** le dispositif de préparation comporte une unité d'alarme et/ou une unité de sortie destinée à fournir en sortie une information à un utilisateur, qui sont réalisées de telle manière qu'une alarme est déclenchée ou une information est fournie en sortie, lorsque le dépassement par le haut ou par le bas d'une plage est détecté.

**12.** Dispositif de préparation selon la revendication 10 ou 11, **caractérisé en ce que** le dispositif de préparation comporte un conduit principal, dans lequel débouche un conduit d'alimentation relié au récipient de bicarbonate et dans lequel débouche un autre conduit d'alimentation relié au récipient de sodium et/ou **en ce que** le conduit principal est relié à un récipient ou à une source d'eau, de préférence d'eau osmosée et/ou **en ce que** la cellule de mesure de conductivité est disposée en aval des deux embouchures des conduits d'alimentation.

**13.** Dispositif de préparation selon l'une des revendications 10 à 12, **caractérisé en ce que** le dispositif de préparation peut être amené en liaison fluidique avec un dialyseur d'un appareil de dialyse, de telle sorte que le dialysat obtenu traverse le côté dialysat du dialyseur et **en ce que** le dispositif de préparation comporte un conduit de dérivation qui contourne le dialyseur de telle sorte que le dialysat obtenu peut contourner temporairement le dialyseur.

**14.** Appareil de dialyse doté d'un dispositif de préparation selon l'une des revendications 10 à 13.

**FIG. 1**

Na- Überwachung                    Bic- Überwachung

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0597817 B1
- US 2014319030 A1
- EP 0311848 A2
- DE 3416057 A1
- US 2016051949 A